(19) Europäisches Patentamt
European Patent Office
Office européen des brevets

(11) **EP 3 272 231 B1**

(12) **EUROPEAN PATENT SPECIFICATION**

(45) Date of publication and mention
of the grant of the patent:
**13.11.2019 Bulletin 2019/46**

(51) Int Cl.:
*A23L 27/10* (2016.01)     *A23L 2/00* (2006.01)
*A23L 2/06* (2006.01)     *A23L 2/56* (2006.01)

(21) Application number: **16764979.7**

(22) Date of filing: **15.03.2016**

(86) International application number:
**PCT/JP2016/058181**

(87) International publication number:
**WO 2016/148149 (22.09.2016 Gazette 2016/38)**

(54) **LIQUID COMPOSITION CONTAINING CITRUS PEEL ESSENTIAL OIL**

FLÜSSIGE ZUSAMMENSETZUNG MIT ÄTHERISCHEM ÖL AUS DER ZITRUSFRUCHTSCHALE

COMPOSITION LIQUIDE CONTENANT DE L'HUILE ESSENTIELLE D'ÉCORCE D'AGRUMES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2015 ES 201530335**

(43) Date of publication of application:
**24.01.2018 Bulletin 2018/04**

(73) Proprietor: **Suntory Holdings Limited
Kita-ku, Osaka-shi
Osaka 530-8203 (JP)**

(72) Inventors:
• **IBUSUKI, Daigo**
**Kawasaki-shi**
**Kanagawa 211-0067 (JP)**
• **FUJIWARA, Masaru**
**Kawasaki-shi, Kanagawa 211-0067 (ES)**
• **YOKOO, Yoshiaki**
**Kawasaki-shi, Kanagawa 211-0067 (JP)**

(74) Representative: **Vossius & Partner
Patentanwälte Rechtsanwälte mbB
Siebertstrasse 3
81675 München (DE)**

(56) References cited:
JP-A- H11 318 379       JP-A- H11 318 379
JP-A- H11 318 379       JP-A- 2000 308 475
JP-A- 2000 350 571       JP-A- 2000 350 571
JP-A- 2008 061 511       JP-A- 2010 088 348
JP-A- 2011 055 797       JP-A- 2011 055 797
JP-A- 2011 055 797

• **ANDREA V ET AL: "Analysis of some Italian lemon liquors (limoncello)", MEDICINAL & AROMATIC PLANTS ABSTRACTS, SCIENTIFIC PUBLISHERS, SCIENTIFIC PUBLISHERS, NEW DELHI - INDIA, vol. 25, no. 6, 1 December 2003 (2003-12-01), XP018007074, ISSN: 0250-4367**
• **MARINA RUSSO ET AL: "Underestimated sources of flavonoids, limonoids and dietary fibre: Availability in lemon's by-products", JOURNAL OF FUNCTIONAL FOODS, vol. 9, 1 July 2014 (2014-07-01), pages 18-26, XP055503476, NL ISSN: 1756-4646, DOI: 10.1016/j.jff.2014.04.004**
• **YASUHIRO OKUBO: 'Production Technology and Feature of Functional Emulsion, using Food Emulsifier' THE FOOD INDUSTRY vol. 56, 01 January 2013, pages 66 - 73, XP009505923**
• **YOSHIAKI MIYAKE ET AL.: 'Flavonoid, Phenylpropanoid and Coumarins Characteristics in Lemons from the Bonin Island s (Kikuchi Lemons' JOURNAL OF THE JAPANESE SOCIETY FOR FOOD SCIENCE AND TECHNOLOGY vol. 60, no. 1, 2013, pages 38 - 42, XP055312042**

**Description**

TECHNICAL FIELD

The present invention relates to liquid compositions containing essential oils of citrus fruits. More particularly, the present invention relates to liquid compositions that comprise essential oils from peel and specified amounts of citropten and hesperidin and which are capable of inclusion within drinks. The invention pertains to the appended claims.

BACKGROUND ART

Some citrus fruits are high in acidity and unsuitable for eating raw but the distinctive fragrance of essential oil components contained in the peel is appreciated. In particular, the juice of citrus fruits having high acidity and a strong aroma of their essential oil (flavorful acid citrus fruits), including lemons and limes, is added to seasonings and drinks. The juice of citrus fruits is usually produced by the in-line method. This method is known to provide juice having a fresh and pleasant scent characteristic of citrus fruits since essential oil from peels is included in the juice in an amount of about 0.01-0.03%, and leftover peels, segment membranes and seeds that impart an undesirable aroma and flavor to the juice are separated from the juice during squeezing. However, the aroma components of citrus fruits are not stable and their quality significantly drops during thermal pasteurization, storage, or distribution: the fresh and pleasant scent is decreased, and deteriorated smells such as chemical-like or oxidized odor occur. To deal with this problem, there has been proposed a method for stably retaining the aroma of flavorful acid citrus fruits juice comprising squeezing citrus fruits and immediately adding a pH adjuster thereto to control the pH of the juice to 3.5 to 7.0 (Patent Document 1).
In the case of producing drinks from high acidity fruits such as flavorful acid citrus fruits, their juice cannot be included in large quantities since the resulting products become so high in acidity that they are not easy to drink. To deal with this problem, it has been proposed to reduce the acidity or bitterness by adding a hesperidin glycoside or the mixture of a hesperidin glycoside with hesperidin to citrus fruits juice (Patent Document 2). Also, there has been proposed a method of increasing a fruit juice-like aroma and flavor by adding citropten to drinks (Patent document 3).

CITATION LIST

PATENT DOCUMENTS

Patent Document 1: JP 2000-308475A

Patent Document 2: JP HI 1-318379 A

Patent Document 3: JP 2011-55797 A

SUMMARY OF INVENTION

TECHNICAL PROBLEM

An object of the present invention is to provide liquid compositions that have abundant aromas reminiscent of natural citrus fruits and which stably retain the aromas after a long-term storage.

SOLUTION TO PROBLEM

The present inventors made intensive studies focusing on the concentrations of citropten and hesperidin in fruit juice, and found that an effect of citropten of enhancing a fruit juice-like aroma and flavor was further increased by combining hesperidin with citropten. However, in the case of juice having high acidity, it turned out that a deteriorated smell tended to occur in some concentrations of citropten and hesperidin, and an aroma characteristic of citrus fruits tended to be lost after a long-term storage. As a result of extensive research to solve the problem the inventors found that a liquid composition that was obtained by crushing the peel within water had a fresh and pleasant fragrance characteristic of citrus fruits even though it did not contain fruit juice. The inventors further found that, by more precisely controlling the essential oil content, the acidity, and the citropten and hesperidin concentrations of flavorful acid citrus fruits and other citrus fruits, a change in aroma can be prevented at a high level even in the case of long-term storage, and thereby completed the present invention.

Specifically, the liquid composition:

> comprises an essential oil from peel of one or more citrus fruits, citropten, and hesperidin; wherein
> the content of the essential oil from peel is in the range of 0.2-3.5% by volume based on the total amount of the composition;
> the concentration of the citropten with respect to acidity is in the range of 5-20 mg/kg/Acid; and
> the concentration of the hesperidin with respect to acidity is 300 mg/kg/Acid or more; and wherein the liquid composition comprises 1% by weight or less of ethanol.

The above-described citropten and hesperidin may be derived from one or more citrus fruits. The above-described liquid composition may comprise the essential oil and the citropten and hesperidin derived from one or more citrus fruits. Compared to conventional types of fruit juice (including comminuted juice), the liquid composition has higher concentrations of the citropten and hesperidin. It should be noted here that in the present invention "mg/kg/Acid" as the unit of concentrations is obtained by dividing the concentration of each component in the composition (mg/kg) by the acidity of the composition. The acidity as used herein is determined by first measuring the content of organic acids in a liquid by neutralization titration with sodium hydroxide, calculating the same in terms of citric acid, and expressing the converted value in percentage. The present inventors found that by incorporating citropten and hesperidin in the liquid composition at certain ranges of concentrations, an aroma and flavor reminiscent of natural fruits was significantly enhanced and was stably retained after a long-term storage.

The liquid composition preferably has an acidity of 2.0 or less and has a by far lower acidity than conventional types of fruit juice. By limiting the acidity to a lower level, the decrease or deterioration of aroma can be suppressed that occurs during the process of thermal pasteurization or a long-term storage. Conventional types of juice are limited in amount of inclusion within drinks since such juice is high in acidity and, if it is included in a large quantity, the resulting products become so sour that they are not easy to drink. On the other hand, a liquid composition with low acidity can be incorporated in drinks in a large quantity without excessively increasing the acidity of the drinks.

The above-stated liquid composition preferably has a concentration of rutin with respect to acidity of 50 mg/kg/Acid or less. By limiting the rutin concentration to lie within a certain range, long lasting, irritating bitterness can be suppressed and a natural fruit-like aroma and flavor from citropten becomes more perceivable.

BRIEF DESCRIPTION OF DRAWINGS

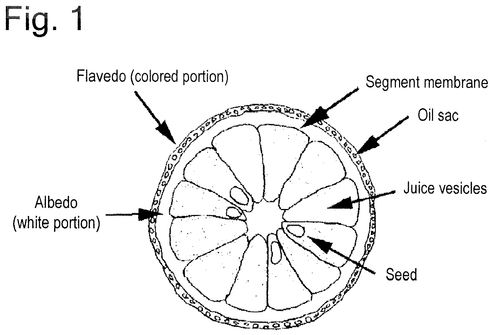

Fig. 1 is a cross section of a citrus fruit.

DESCRIPTION OF EMBODIMENTS

<Essential oils from peel>

The composition of the present invention contains essential oils from peel of citrus fruits in amounts ranging from 0.2 to 3.5% by volume based on the total amount of the composition. The essential oils are a class of fragrant compounds that are mainly collected from the peels of citrus fruits. The essential oils are based on terpene compounds such as monoterpenes and sesquiterpenes and differ from oils and fats that are based on glycerides. Although the essential oils from peel to be used in the present invention may be obtained by any of known methods, it is preferable to use the essential oil components that are obtained by expression.

The term "expression" as used herein refers to a method in which a physical force is applied to the peel of a fruit so that an essential oil is obtained from oil cells present in the colored portion of the peel. In a known example of the expression process, the peel is mechanically bruised to rupture oil sacs, from which an essential oil is extracted (Florida Citrus Oils, Kesterson, et al., Technical Bulletin 749, December 1971, pp15-20.) As will be described later, the peel containing an essential oil may be emulsified under crushing in water and this method is also included in the expression process. The citrus peel includes a dark colored flavedo portion and a white fibrous albedo portion, with the flavedo containing a lot of oil sacs containing a large amount of essential oil (see Fig. 1 quoted from "Kajitsu no Jiten (Dictionary of Fruits)", published by Asakura Shoten, 2008, p. 198.) In the present invention, the peel, or the flavedo in particular, is collected and by emulsifying the oil sac-containing peel as it is crushed in water, a liquid composition can be obtained that contains the essential oil from peel. In the process, the peel or flavedo is preferably collected in such a way that the oil sacs are kept intact as much as possible until they are ruptured within water. By maintaining the oil sacs intact until they are emulsified in water, the essential oil in the oil sacs can be prevented from making direct contact with oxygen to reduce the possibility that the aroma components in the essential oil may deteriorate upon oxidation. The thus obtained essential oil is advantageous in that it is less susceptible to heat and oxygen and that, therefore, the aroma components will experience less deterioration. When the cold press method or other conventional technique for obtaining an essential oil from peel is employed, it is necessary to take an essential oil out of oil sacs before the process completes, so its aroma components may deteriorate under direct action of oxygen.

Examples of the citrus fruits to be used for obtaining essential oils from peel include those having juice with an acidity of at least 0.40%. Fruits that are advantageous for the present invention include not only what are called "flavorful acid citrus fruits," such as Citrus limon, Citrus aurantifolia, Citrus sudachi, Citrus sphaerocarpa, Citrus depressa, and Citrus junos, but also oranges (Citrus sinensis) (Valencia orange, Navel orange, Blood orange, etc.); grapefruits (Citrus paradisi) (marsh, ruby, etc.); other citrus (Citrus natsudaidai, Citrus hassaku, Citrus tamurana, Citrus grandis x paradisi (Oroblanco), Citrus reticulata cv. Dekopon, etc.); tangors (Citrus iyo, Citrus tankan, Citrus unshiu x sinensis, Citrus sinensis (Harumi), etc.); tangelos (Citrus x tangelo cv. Seminole, Citrus sinensis (Minneola), etc.); buntans (Citrus maxima, Citrus grandis (Banpeiyu), etc.); and Satsuma mandarins (mandarin orange, Citrus kinokuni, Citrus unshiu, Citrus reticulata cv. Ponkan, Citrus tachibana, etc.). The genus Fortunella includes Fortunella crassifolia, Fortunella japonica, Fortunella margarita, etc.

The concentration of the essential oil from peel in the liquid composition ranges from 0.2 to 3.5% by volume based on the total amount of the composition. Preferably, it ranges from 0.3 to 3.0% by volume, more preferably from 0.4 to 2.5% by volume, even more preferably from 0.4 to 2.0% by volume, and particularly preferably from 0.4 to 1.5% by volume. The concentration of the essential oil from peel in the composition can be measured with an essential oil testing apparatus, or a distillation system using an essential oil quantifying device, as will be described below in the Examples.

<Citropten>

The composition of the present invention comprises citropten in addition to the essential oil from peel. Citropten (5,7-dimethoxycoumarin, molecular formula $C_{11}H_{10}O_4$) is a component that has slight bitterness found in the peel or oil in the peel of citrus fruits.

The concentration of citropten in the composition is in the range of 5-20 mg/kg/Acid, preferably in the range of 8-18 mg/kg/Acid. It should be noted here the unit "mg/kg/Acid" refers to the concentration of citropten with respect to acidity and derives from the concentration of citropten in the composition (mg/kg) as a result of dividing it by the acidity of the composition. The acidity as used herein is determined by first measuring the content of organic acids in the composition by neutralization titration with sodium hydroxide, calculating the same in terms of citric acid, and expressing the converted value in percentage. The concentration of citropten in the liquid composition of the present invention is generally higher than those in conventional types of fruit juice (including comminuted juice).

The citropten contained in the liquid composition of the present invention is preferably derived from citrus fruits, particularly preferably from flavorful acid citrus fruits, although this is not the sole example of citropten that can be used in the present invention. By using the peel-derived citropten and combining it with the essential oil from peel, there would be obtained a composition whose aroma is even closer to that of natural citrus fruits and which has high storage stability.

The concentration of citropten to be used in the present invention can be adjusted by adding a commercially available citropten into the composition. Alternatively, as will be understood from the method to be described later, the same result can be obtained by emulsifying the essential oil-containing peel or flavedo as they are crushed within water so that the peel-derived citropten is extracted into the liquid composition. The citropten concentration in the liquid composition can be measured by use of LC-MS, as will be described later in the Examples.

<Hesperidin>

The composition of the present invention comprises hesperidin in addition to the essential oil from peel and citropten. Hesperidin (molecular formula $C_{28}H_{34}O_{15}$) is a type of flavanone glycosides and is a component found in the peel, segment membrane, or the like in citrus fruits.

The concentration of hesperidin in the composition is preferably 300 mg/kg/Acid or more, more preferably in the range of 300-800 mg/kg/Acid, even more preferably 350-700 mg/kg/Acid. The present inventors found that, by adjusting the concentrations of citropten and hesperidin with regard to acidity, it is possible to impart a natural fruit-like aroma and flavor to the composition and improve its storage stability. It has already been known that citropten has moderate bitterness and astringency and, if being added to drinks having zero or low content of fruit juice, citropten imparts a fruit juice-like aroma and flavor to drinks (JP 2011-55795 A); however it has never been known that this action can be enhanced by combining hesperidin contained at a certain range of concentration with the citropten. Hesperidin has no taste and smell, and thus it comes as a surprise that the action of citropten can be enhanced by hesperidin.

The hesperidin contained in the liquid composition of the present invention is preferably derived from one or more citrus fruits, particularly preferably from one or more flavorful acid citrus fruits, although this is not the sole example of hesperidin that can be used in the present invention. By using the peel-derived hesperidin and combining it with the essential oil from peel, there would be obtained a composition whose aroma is even closer to that of natural citrus fruits.

The concentration of hesperidin to be used in the present invention can be adjusted by adding a commercially available hesperidin into the composition. Alternatively, as will be understood from the method to be described later, the same result can be obtained by emulsifying the essential oil-containing peel or flavedo as they are crushed within water so that the peel-derived hesperidin is extracted into the liquid composition. The hesperidin concentration in the liquid composition can be measured by use of LC-MS, as will be described later in the Examples.

<Rutin>

The concentration of rutin in the composition of the present invention is preferably 50 mg/kg/Acid or less, more preferably 5-50 mg/kg/Acid, even more preferably in the range of 10-40 mg/kg/Acid. By incorporating rutin in the liquid composition at a certain range of concentration, an action of hesperidin of enhancing the aroma and flavor of citropten can be increased synergistically. Rutin (quercetin-3-glucoside, molecular formula $C_{27}H_{30}O_{16}$) is a type of flavone glycosides and is known to be contained in soba (Fagopyrum exculentum), asparagus, etc., and it is also known to be contained in the peel and others of citrus fruits. The present inventors found that by adjusting the concentration of rutin in the composition containing essential oil from peel, citropten, and hesperidin to lie within a certain range, irritating and long-lasting intense bitterness specific to flavorful acid citrus fruits can be suppressed. Rutin has no taste or smell, and thus it comes as a surprise that bitterness can be suppressed by limiting the amount of rutin.

The rutin contained in the liquid composition of the present invention is preferably derived from one or more citrus fruits although this is not the sole example of rutin that can be used in the present invention. The concentration of rutin can be adjusted by adding a certain amount of commercially available rutin into the composition containing essential oil from peel, citropten, and hesperidin, or it can also be adjusted by addition or removal of rutin to or from the liquid composition obtained by use of citrus fruits. As will be understood from the method to be described later, the liquid composition may be obtained by emulsifying the essential oil-containing peel or flavedo as they are crushed within water so that the peel-derived essential oil, citropten, and hesperidin are extracted into the liquid composition. In this case, usually, a large amount of rutin is also extracted into the liquid composition, so an appropriate amount of rutin needs to be removed to limit the rutin concentration to lie within the above-mentioned ranges. In this regard, the present inventors have found a convenient method for limiting the rutin concentration in the composition to lie within the above-mentioned ranges, namely, they have found that the concentration of rutin can be reduced by removing the super surface layer of peel very thinly (at such a thickness that oil sacs are kept intact) before crushing the peel or flavedo within water. Although it was already known that rutin is contained in the peel of citrus fruits, the present inventors first found out that the content of rutin is high, in particular, near the outer surface of the peel. The rutin concentration in the liquid composition can be measured by use of LC-MS, as will be described later in the Examples.

<Acidity>

If the essential oils of citrus fruits are placed under high acidity conditions, their fresh and pleasant aroma will readily be decreased to give a chemical-like deteriorated smell. The composition of the present invention, being reduced in acidity, can be used as an aroma composition that is more natural and fresh with less deterioration. The composition of the present invention has an acidity (as calculated for citric acid) of 2.0% or less, preferably 0.1-1.5%, more preferably 0.2-1.2%, and even more preferably 0.2-1.0%. Unlike conventional types of juice (including comminuted fruit juice), the composition of the present invention contains no components of fruit juice, so even if high acidity fruits are used, the acidity of the composition can be reduced to lower levels.

Further, if the composition of the present invention has a lower degrees Brix, an aroma composition that will undergo even less deterioration can be obtained. The composition of the present invention preferably has °Brix of 25% or less, more preferably 20% or less, even more preferably 15% or less, and particularly preferably 1-10%.

<Liquid composition>

The composition of the present invention is in a liquid state at room temperature under atmospheric pressure. It preferably comprises water as the main solvent. The composition comprises 1% by weight or less of ethanol, more preferably no ethanol.

The liquid composition of the present invention preferably has a pH of less than 5, more preferably, less than 4, yet more preferably less than 3.5. The low pH condition is advantageous for suppressing the growth of spoilage microorganisms.

As will be described later in a more specific way, one possible method for producing the liquid composition of the present invention comprises the following procedure: the peel or flavedo having oil sacs containing an essential oil is emulsified as it is crushed in water, whereupon the essential oil are taken out of the oil sacs into the water and the citropten and hesperidin are extracted from the peel into the water. As a result, a liquid flavoring characterized by "no food additives used" can be produced. By the term "no additives used" is meant that none of the additives on the "List of Products on the Registry of Existing Additives" specified in the Japan Food Sanitation Act have been "externally" added. The liquid composition which solely consists of the fruit-derived components and water has a natural fruit-like aroma and flavor and is preferred because the effects of the present invention are developed more markedly.

The liquid composition of the present invention may be incorporated into foods and drinks for the purpose of flavoring them. In the case of drinks, for example, the composition may be incorporated at concentrations varying from 0.1 to 15% by weight, preferably from 0.5 to 10% by weight, depending on the aroma and flavor to be imparted. Being an aqueous composition that is low in either acidity alone or both acidity and degrees Brix and which is relatively high in the content of essential oil from peel, the liquid composition of the present invention has an advantage in that it can be incorporated into drinks in large enough amounts to ensure that the aroma components of the essential oil from peel are amply contained in the drinks. Particularly in the case of producing drinks with the taste of high acidity citrus fruit, the aroma of the fruit can be reproduced within the drinks. The liquid composition of the present invention can also be used to impart the flavor of fruit to black tea and other tea beverages. The drinks in which the liquid composition of the present invention may be used are not particularly limited and may include various types such as alcoholic beverages, alcohol-free beverages, carbonated drinks, juice-containing drinks, and tea-based drinks. It is worth particular mention that if the liquid composition does not contain alcohol (ethanol), it can advantageously be used to flavor alcohol-free drinks. In particular, it can advantageously be used to flavor drinks (non-alcohol drinks) of low juice content which is in the range of about 1-30% by weight, preferably 1-20% by weight, and more preferably 1-10% by weight, and this enables the manufacture of drinks that reproduce the fragrance of natural fruits. By using the liquid composition of the present invention, it is also possible to manufacture drinks that contain no synthetic additives such as synthetic flavors and surfactants.

<Production method>

The liquid composition of the present invention can be produced by adjusting the essential oil from peel, the amounts of citropten and hesperidin, and acidity. If desired, the composition can be produced using only a fruit or fruits and water in accordance with the method described below, which is given here for illustrative purposes only and is not the sole example that can be employed. The liquid composition that solely consists of fruit-derived components and water presents an aroma more like a natural fruit and, hence, is preferred.

First, the peel is collected from a citrus fruit by a commonly employed technique. The collected peel consists of a dark colored flavedo portion having oil sacs and a white fibrous albedo portion. Containing no oil sacs, the albedo portion of the peel has less aroma components than the flavedo portion does; in addition, depending on the fruit from which it is derived, the albedo portion may present a bitter taste, so the flavedo portion may be collected after most of the albedo portion has been removed from the peel. In this process, a small amount of the albedo portion may become included in the flavedo portion. In the process of collecting the peel or flavedo, care is preferably taken to ensure that the oil sacs in the flavedo are least destroyed. By not destroying the oil sacs, the essential oil contained in the oil sacs can be protected from oxidative deterioration.

The collected peel or flavedo is then mixed with water. The mixing ratio of water to peel (by weight) is preferably in the range of from about 0.5:1 to about 2.5:1, more preferably from about 0.6:1 to about 1.8:1, even more preferably from about 0.7:1 to about 1.5:1, and particularly preferably from about 0.7:1 to about 1:1. After mixing the peel with water, an apparatus such as a mixer or homogenizer that is capable of dispersing the peel in water while shredding it into pieces may be used to form an emulsion in water of the essential oil contained in the oil sacs in the peel and to extract the citropten and hesperidin from the peel into water. If the peel or flavedo collected with care being taken to keep the oil sacs intact as much as possible is mixed with water and the essential oil is emulsified while rupturing the oil sacs within water, direct contact of the essential oil with the atmosphere can be avoided to reduce its deterioration. From the resulting mixture of water and the disrupted peel, the solids content is centrifugally or otherwise removed to give a liquid composition. The thus obtained liquid composition solely consists of water and the fruit components and, in the absence of any off-flavors due to components other than the fruit, it presents an aroma and flavor resembling those of natural fruit. As a further advantage, during the production of the liquid composition, since the essential oils from peel does not directly contact with atmosphere (oxygen), the aroma components will undergo less oxidative deterioration and the produced composition presents a fresh aroma and flavor.

After the collection of the peel or flavedo, before mixing it with water, the super surface layer of the peel or flavedo may be removed by slicing the peel thinly enough not to rupture oil sacs in flavedo. Flavedo has oil sac tissue that collects essential oil and there exist a countless number of recessed dots on the fruit surface of flavedo. The super surface layer of flavedo refers to an extreme surface layer having a thickness such that the fruit surface with recessed dots would be smoothed if this layer was removed, which means the thickness is usually within 1 mm from the outer surface of the peel, preferably within 0.7 mm, although varying with types of fruit. The inventors have found that, by removing the super surface layer of the peel and then crushing the peel within water, the rutin content can be significantly reduced compared to a case where the super surface layer has not been removed. A liquid composition in which the rutin concentration is limited to lie within a certain range not only has an aroma and flavor reminiscent of a natural fruit enhanced by citropten and hesperidin but also is reduced in long-lasting and irritating bitterness, hence, is preferred.

EXAMPLES

On the following pages, several examples of the present invention are given but it should be understood that the present invention is by no means limited to these Examples.

(1) Measurement of degrees Brix

Measurement of degrees Brix (%) (°Brix: %) was performed with a digital refractometer (manufactured by ATAGO CO., LTD.; Model No. RX-5000α) at 20°C.

(2) Measurement of acidity

Ten grams of a liquid composition was diluted to a prescribed volume, thereby making a test solution. A given amount of the test solution was titrated with a 0.1 mol/L sodium hydroxide standard solution using phenolphthalein as a pH indicator and the titratable acidity was calculated by the following formula:

$$\text{Acidity (\%)} = K \times (T - B) \times F \times (100/A) \times (1/W) \times 100$$

K: calculated for citric acid = 0.0064
T: the amount of 0.1 mol/L sodium hydroxide solution used for titration (ml)
B: the amount of 0.1 mol/L sodium hydroxide solution used for titration in the same amount of water (ml)
F: the factor of 0.1 mol/L sodium hydroxide solution
A: the volume of sample taken for titration (ml)
W: the weight of sample taken for preparation (g).

(3) Measurement of the essential oil's content

To measure the essential oil's content in the composition, an essential oil quantifying apparatus was used. A round-bottom flask equipped with a condenser capable of trapping the essential oil was charged with 100 mL of the liquid composition, 2 L of distilled water, and boiling stones; atmospheric distillation was performed under heating at about 100°C for an hour and the amount of the essential oil (mL) collecting in the trap tube was measured to calculate the content of the essential oil.

(4) Measurement of citropten

(Preparation of samples for analysis)

Samples for analysis were prepared by the following methods. First, 10 g of the liquid composition was weighed in a centrifugal glass tube (A). Note that when the liquid composition had a degrees Brix of 10% or more, 5 g was weighed; in the case of 20% or more, 2.5 g was weighed; and in the case of 30% or more, 1 g was weighed; in either case, the weighed liquid composition was diluted to 10 mL with distilled water for liquid chromatography. Subsequently, 20 mL of ethanol for liquid chromatography was added and the mixture was vigorously agitated with a vortex mixer for one minute or longer. When high viscosity prevented effective mixing, vigorous manual shaking was optionally performed. The intimate mixture was subjected to a centrifuge (1620 G x 30 min at 20°C) and the supernatant was transferred into another centrifugal glass tube (B). To the precipitate, 20 mL of ethanol for liquid chromatography was added and after breaking the solids loose enough with a suitable device such as a dispensing spoon, the mixture was vigorously agitated with a vortex mixer for one minute or longer. After centrifugation with a centrifuge (1620 G x 30 min at 20°C), the supernatant was charged into the centrifugal tube (B). The collected supernatants in the centrifugal tube (B) were further centrifuged (1620 G x 30 min at 20°C) and the resulting supernatant was transferred into a 50-mL measuring flask and diluted with ethanol to the marked line. The well mixed supernatant was further diluted 10-fold with ethanol for liquid chromatography and passed through a preliminarily ethanol-washed PTFE filter (product of Toyo Roshi Kaisha, LTD; ADVANTEC DISMIC-25HP 25HP020AN, with a pore size of 0.20 μm and a diameter of 25 mm) to prepare samples for analysis.

(Conditions for LC separation)

HPLC apparatus: Agilent 1290 Series (product of Agilent technologies Inc.; equipped with feed pump, G4220A; auto-sampler, G4226A (with thermostat G1330B); column oven, G1316C; and DAD detector, G4212A)
Column: Phenomenex KINETEX C18 100Å (particle size, 1.3 μm; inside diameter, 2.1 mm x 50 mm x two columns coupled in series (product of Phenomenex Company)
Mobile phase A: 0.1% aqueous solution of formic acid
Mobile phase B: acetonitrile
Flow rate: 0.4 mL/min
Density gradient conditions: 0.0-1.0 min (5% B)→29.5-31.5 min (100% B), with 4.5 min equilibration by the initial mobile phase
Column temperature: 40°C
Sample injection: injected in a volume of 1.0 μL
Sample charge into mass spectrometer: 2.0-31.99 min

(Conditions for mass spectroscopy)

Mass spectrometer: Q Exactive (product of Thermo Fisher Scientific Company)
Ionization method: HESI, positive mode

Conditions of ionization chamber:

Sheath gas flow rate,50;
Aux gas flow rate, 10;
Sweep gas flow rate, 0;
Spray voltage, 2.50 kV;
Capilary temp, 350°C;
Aux gas heater temp, 300°C

Detection conditions:

Resolution, 70000;
AGC Target, 1e6;
Maximum IT, 100 ms;
Scan Range, 100 to 1000 m/z

Analysis condition:

The peak detection time of citropten was 10.37 min in an extracted ion chromatogram for 207.0647-207.0667 m/z. For each sample, an elusion time should be confirmed using a standard sample.

(Quantification method)

Standard samples were purchased from Alfa Aesar Company. At least three standard sample solutions of different concentrations were used and quantification was performed by the absolute calibration method based on the peak areas obtained. In the case of measurements that turned out to give values that were outside the ranges of calibration curves, the factor of dilution with ethanol at the final stage of analysis sample preparation was appropriately adjusted to perform another measurement.

(5) Hesperidin measurement

Samples for analysis that were prepared by the same methods as that used for citropten were measured on the following conditions.

(Conditions for LC separation)

HPLC apparatus: Nexera XR Series (product of Shimadzu Corporation; equipped with system controller, CBM-20A; feed pump, LC-20ADXR; on-line degasser, DGU-20A3; auto-sampler, SIL-20ACXR; column oven, CTO-20A; and UV/VIS detector, SPD-20A) Column: CAPCELL CORE AQ (particle size, 2.7 $\mu$m; inside diameter, 2.1 mm x 150 mm; product of Shiseido Company, Limited)
Mobile phase A: 0.1% aqueous solution of formic acid
Mobile phase B: acetonitrile
Flow rate: 0.6 mL/min
Density gradient conditions: 0.0-0.5 min (15% B)→6.0 min (25% B), with 10.0 min (75% B) → 10.1-11.0 min (100% B), with 3.0 min equilibration by the initial mobile phase
Column temperature: 40°C
Sample injection: injected in a volume of 2.0 $\mu$L
Sample charge into mass spectrometer: 1.8-11.0 min

(Conditions for mass spectroscopy)

Mass spectrometer: 4000 Q TRAP (product of AB Sciex)
Ionization method: ESI (Turbo Spray), negative mode
Conditions of ionization chamber: CUR, 10; IS, -4500; TEM, 650; GS1, 80; GS2, 60; ihe, ON; CAD, Medium
Detection method: MRM mode
Detection conditions: (Q1→Q3, DP, CE, CXP, EP): Hesperidin (609.2→301.1, 1, -76, -50, -11, -10)
Peak detection time: Being subject to confirmation with standard samples, the following data may be given as a guide.

Hesperidin (4.67 min)

(Quantification method)

Standard samples were purchased from Wako Pure Chemical Industries, Ltd. At least three standard sample solutions of different concentrations were used and quantification was performed by the absolute calibration method based on the peak areas obtained. In the case of measurements that turned out to give values that were outside the ranges of calibration curves, the factor of dilution with ethanol at the final stage of analysis sample preparation was appropriately adjusted to perform another measurement.

(6) Rutin measurement

Samples for analysis that were prepared by the same methods as that used for hesperidin were measured on the following conditions.
(Conditions for LC separation): The same as for hesperidin is applicable.

(Conditions for mass spectroscopy)

Mass spectrometer: 4000 Q TRAP (product of AB Sciex)
Ionization method: ESI (Turbo Spray), positive mode
Conditions of ionization chamber: CUR, 10; IS, 5500; TEM, 650; GS1, 80; GS2, 60; ihe, ON; CAD, Medium
Detection method: MRM mode
Detection conditions: (Q1→Q3, DP, CE, CXP, EP): Rutin (611.2→303.1, 76, 25, 12, 10)

Peak detection time: Being subject to confirmation with standard samples, the following data may be given as a guide.

Rutin (3.01 min)

(Quantification method)

Standard samples were purchased from Wako Pure Chemical Industries, Ltd. At least three standard sample solutions of different concentrations were used and quantification was performed by the absolute calibration method based on the peak areas obtained. In the case of measurements that turned out to give values that were outside the ranges of calibration curves, the factor of dilution with ethanol at the final stage of analysis sample preparation was appropriately adjusted to perform another measurement.

<Example 1>

(1) Production of lemon aroma composition

Peel was collected from lemons (FINO) and most of the albedo was removed from the peel. During the peel collection and albedo removal, care was taken to minimize possible damage to the oil sacs in the peel. The remaining lemon's flavedo was mixed with water at a weight ratio of 1:1 and the mixture was milled with a commercial juice mixer, with care being taken to ensure that the mixture would not have a paste-like consistency; after stirring at room temperature for 30 minutes, the mixture was passed through a 40-mesh strainer to effect solid-liquid separation. The liquid phase was thereafter homogenized at 0.2 MPa and the insoluble solids were removed from the resulting suspension by centrifugation (6000 G x 5 min) and then pasteurized by heating at 90°C for one minute to thereby prepare a liquid composition (Invention Product 1). Invention Product 1 was compared with three types of commercial comminuted lemon juice (Comparative Examples 1-3) and the results are shown in Table 2 below. The various components were measured by the methods described above.

(2) Sensory evaluation

To solutions adjusted with sucrose and citric acid anhydride to °Brix 10 and acidity 0.15%, Invention Product 1 prepared in (1) above was added at a concentration of 10 g/L and the sample was then filled into a bottle and pasteurized by heating at 85°C for 5 minutes to produce a bottled drink with lemon taste. Also, the following drinks were produced: a drink containing Comparative Example 1 at a concentration of 2.4 g/L, a drink containing Comparative Example 2 at a concentration of 2.5 g/L, and a drink containing Comparative Example 3 at a concentration of 10 g/L. The obtained drinks were evaluated for their flavor. The evaluated flavors were the intensity of a natural fresh aroma and the intensity of a deteriorated smell, and the grading system was on a 5-point scale using the criteria shown in Table 1 below. The results are shown in Table 2. Invention Product 1, although being subjected to a thermal pasteurization treatment, had a fresh and pleasant aroma resembling that before heating. The aroma was reminiscent of a natural fruit even though the sample did not contain fruit juice. On the other hand, in the commercial comminuted juice (Comparative Examples 1-3), a scent characteristic of citrus fruits was reduced and deteriorated. Further, by use of Invention Product 1 stored in a refrigerator for one month, a lemon taste drink was produced in the same method and was evaluated for flavor. The drink stably retained a scent characteristic of citrus fruits after the storage and hardly emitted a perceivable deteriorated smell.

[Table 1]

| Score | Degree |
|---|---|
| 4 | Perceived ntensely |
| 3 | Perceived considerably |
| 2 | Perceived moderately |
| 1 | Perceived slightly |
| 0 | Not perceived |

[Table 2]

| | Invention Product 1 | Comparative Example 1 | Comparative Example 2 | Comparative Example 3 |
|---|---|---|---|---|
| Degrees Brix (%) | 5.7 | 23.9 | 22.8 | 5.7 |
| Acidity (%) | 0.67 | 11.5 | 8.6 | 2.4 |
| Essential oil content (%) | 1.10 | 1.1 | 1.2 | 0.8 |
| Citropten concentration per acidity (mg/kg/Acid) | 11.2 | 1.1 | 2.0 | 6.1 |
| Hesperidin concentration per acidity (mg/kg/Acid) | 430.3 | 59.0 | 63.3 | 278.9 |
| Rutin concentration per acidity (mg/kg/Acid) | 92.7 | 3.4 | 4.9 | 9.8 |
| Flavor evaluation (Intensity of fresh aroma) | 4 | 2 | 2 | 1 |
| Flavor evaluation (Intensity of deteriorated smell | 0 | 2 | 1 | |
| Flavor evaluation after storage (Intensity of fresh aroma) | 4 | | | |
| Flavor evaluation after storage (Intensity of deteriorated smell) | 1 | | | |

<Example 2>

A liquid composition (Invention Product 2) was obtained by use of the flavedo of the same lemon and the method as used to produce Invention Product 1 in Example 1, except that before mixing the flavedo with water the super surface layer of it was removed by slicing the flavedo thinly enough not to rupture oil sacs in it. In the same methods as described in Example 1, the obtained liquid composition was subjected to the measurement of components and the flavor of the produced lemon taste drink was evaluated. The results are shown in Table 3. It turned out that, by removing the super surface layer of the peel and then crushing the peel within water (Invention Product 2), the rutin content could be significantly reduced compared to a case where the super surface layer was not removed (Invention Product 1). The liquid composition in which the rutin concentration was limited to lie within a certain range not only had an aroma and flavor reminiscent of a natural fruit enhanced by citropten and hesperidin but also was reduced in irritating bitterness that lasts long into the aftertaste and, hence, was preferred. This preferable aroma and flavor stably retained after storage.

[Table 3]

| | Invention Product 1 | Invention Product 2 |
|---|---|---|
| Degrees Brix (%) | 5.7 | 4.2 |
| Acidity (%) | 0.67 | 0.38 |
| Essential oil content (%) | 1.10 | 0.45 |
| Citropten concentration per acidity (mg/kg/Acid) | 11.2 | 8.7 |
| Hesperidin concentration per acidity (mg/kg/ Acid) | 430.3 | 602.0 |
| Rutin concentration per acidity (mg/kg/Acid) | 92.7 | 47.1 |

<Example 3>

Lemon aroma compositions were produced by use of a variety of lemons in the same method as used to produce Invention Product 2 in Example 2. In the same methods as described in Example 1, the obtained liquid compositions were subjected to the measurement of components and the produced lemon taste drinks were evaluated for their flavor. The results are shown in Table 4. Invention Products 3-12 in which acidities and concentrations of citropten, hesperidin, and rutin were adjusted to lie within certain ranges had a fresh and pleasant aroma characteristic of flavorful acid citrus fruits.

[Table 4]

|  | Invention product 3 | Invention product 4 | Invention product 5 | Invention product 6 | Invention product 7 |
|---|---|---|---|---|---|
| Degrees Brix (%) | 4.8 | 4.6 | 4.1 | 4.5 | 4.5 |
| Acidity (%) | 0.63 | 0.63 | 0.57 | 0.67 | 0.57 |
| Essential oil content (%) | 0.70 | 0.65 | 0.45 | 0.55 | 0.80 |
| Citropten concentration per acidity (mg/kg/Acid) | 15.0 | 13.2 | 11.1 | 5.5 | 7.9 |
| Hesperidin concentration per acidity (mg/kg/Acid) | 482.8 | 451.9 | 447.8 | 350.4 | 494.4 |
| Rutin concentration per acidity (mg/kg/Acid) | 20.7 | 19.8 | 18.6 | 11.4 | 18.4 |

|  | Invention product 8 | Invention product 9 | Invention product 10 | Invention product 11 | Invention product 12 |
|---|---|---|---|---|---|
| Degrees Brix (%) | 4.1 | 3.9 | 4.1 | 3.9 | 4.2 |
| Acidity (%) | 0.52 | 0.36 | 0.60 | 0.58 | 0.60 |
| Essential oil content (%) | 0.45 | 0.45 | 0.40 | 0.38 | 0.50 |
| Citropten concentration per acidity (mg/kg/Acid) | 7.4 | 7.9 | 8.7 | 9.2 | 7.1 |
| Hesperidin concentration per acidity (mg/kg/Acid) | 588.2 | 486.4 | 602.0 | 577.6 | 545.6 |
| Rutin concentration per acidity (mg/kg/Acid) | 17.3 | 39.0 | 47.1 | 17.2 | 13.9 |

**Claims**

1. A liquid composition comprising an essential oil from peel of a citrus fruit, citropten, and hesperidin,
   wherein a content of the essential oil from peel is in a range of 0.2-3.5% by volume of the total amount of the composition;
   wherein a concentration of the citropten with respect to acidity is in a range of 5-20 mg/kg/Acid;
   wherein a concentration of the hesperidin with respect to acidity is 300 mg/kg/Acid or more; and wherein the liquid composition comprises 1 % by weight or less of ethanol.

2. The liquid composition according to claim 1, wherein the citropten and hesperidin are derived from peel of one or more of citrus fruits.

3. The liquid composition according to claim 1 or 2 further comprising rutin, wherein a concentration of rutin with respect to acidity is 50 mg/kg/Acid or less.

4. The liquid composition according to claim 3, wherein the rutin is derived from peel of one or more of citrus fruits.

5. The liquid composition according to any one of claims 1 to 4 which has an acidity of 2.0% or less.

**6.** A drink containing the liquid composition according to any one of claims 1 to 5 at a concentration in a range of 0.1-15% by weight based on the total weight of the drink.

**Patentansprüche**

**1.** Eine flüssige Zusammensetzung, umfassend ein ätherisches Öl aus der Schale einer Zitrusfrucht, Citropten und Hesperidin,
wobei ein Gehalt des ätherischen Öls aus der Schale in einem Bereich von 0,2 - 3,5 Volumen-% der Gesamtmenge der Zusammensetzung liegt;
wobei eine Konzentration des Citroptens, bezogen auf den Säuregehalt, in einem Bereich von 5 - 20 mg/kg/Säure liegt;
wobei eine Konzentration des Hesperidins, bezogen auf den Säuregehalt, 300 mg/kg/Säure oder mehr beträgt; und
wobei die flüssige Zusammensetzung 1 Gew.-% oder weniger Ethanol umfasst.

**2.** Die flüssige Zusammensetzung nach Anspruch 1, wobei das Citropten und Hesperidin von der Schale einer oder mehrerer Zitrusfrüchte stammen.

**3.** Die flüssige Zusammensetzung nach Anspruch 1 oder 2, ferner umfassend Rutin, wobei eine Konzentration von Rutin, bezogen auf den Säuregehalt, 50 mg/kg/Säure oder weniger beträgt.

**4.** Die flüssige Zusammensetzung nach Anspruch 3, wobei das Rutin von der Schale einer oder mehrerer Zitrusfrüchte stammt.

**5.** Die flüssige Zusammensetzung nach einem der Ansprüche 1 bis 4, welche einen Säuregehalt von 2,0% oder weniger hat.

**6.** Ein Getränk, welches die flüssige Zusammensetzung nach einem der Ansprüche 1 bis 5 in einer Konzentration im Bereich von 0,1 - 15 Gew.-%, bezogen auf das Gesamtgewicht des Getränks, enthält.

**Revendications**

**1.** Composition liquide comprenant une huile essentielle d'écorce d'agrume, du citroptène et de l'hespéridine,
dans laquelle une teneur de l'huile essentielle d'écorce est dans une plage de 0,2 à 3,5 % en volume de la quantité totale de la composition ;
dans laquelle une concentration du citroptène par rapport à l'acidité est dans une plage de 5 à 20 mg/kg/acide ;
dans laquelle une concentration de l'hespéridine par rapport à l'acidité est de 300 mg/kg/acide ou plus ; et dans laquelle la composition liquide comprend 1 % en poids ou moins d'éthanol.

**2.** Composition liquide selon la revendication 1, dans laquelle le citroptène et l'hespéridine sont dérivés d'écorce d'un ou plusieurs agrumes.

**3.** Composition liquide selon la revendication 1 ou 2 comprenant en outre de la rutine, dans laquelle une concentration de rutine par rapport à l'acidité est de 50 mg/kg/acide ou moins.

**4.** Composition liquide selon la revendication 3, dans laquelle la rutine est dérivée d'écorce d'un ou plusieurs agrumes.

**5.** Composition liquide selon l'une quelconque des revendications 1 à 4 qui a une acidité de 2,0 % ou moins.

**6.** Boisson contenant la composition liquide selon l'une quelconque des revendications 1 à 5 à une concentration dans une plage de 0,1 à 15 % en poids sur la base du poids total de la boisson.

# Fig. 1

Flavedo (colored portion)

Segment membrane

Oil sac

Albedo
(white portion)

Juice vesicles

Seed

## REFERENCES CITED IN THE DESCRIPTION

### Patent documents cited in the description

- JP 2000308475 A
- JP HI1318379 A
- JP 2011055797 A
- JP 2011055795 A

### Non-patent literature cited in the description

- **KESTERSON et al.** Florida Citrus Oils. *Technical Bulletin,* December 1971, vol. 749, 15-20
- Kajitsu no Jiten (Dictionary of Fruits). Asakura Shoten, 2008, 198